(19) Europäisches Patentamt
European Patent Office
Office européen des brevets

(11) **EP 3 139 173 A1**

(12) **EUROPÄISCHE PATENTANMELDUNG**

(43) Veröffentlichungstag:
**08.03.2017 Patentblatt 2017/10**

(51) Int Cl.:
*G01N 33/68* [(2006.01)]     *G01N 33/74* [(2006.01)]

(21) Anmeldenummer: **16189548.7**

(22) Anmeldetag: **03.03.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **03.03.2007 DE 102007010834**

(62) Dokumentnummer(n) der früheren Anmeldung(en) nach Art. 76 EPÜ:
**08734336.4 / 2 122 363**

(71) Anmelder: **B.R.A.H.M.S GmbH**
**16761 Hennigsdorf (DE)**

(72) Erfinder:
• **Bergmann, Andreas**
**12351 Berlin (DE)**

• **Struck, Joachim**
**13465 Berlin (DE)**
• **Morgenthaler, Nils**
**13503 Berlin (DE)**
• **Papassotiriou, Jana**
**verstorben (DE)**
• **Anker, Stefan**
**13053 Berlin (DE)**

(74) Vertreter: **Simandi, Claus**
**Patentanwalt**
**Höhenstrasse 26**
**53773 Hennef / Bonn (DE)**

Bemerkungen:
Diese Anmeldung ist am 19.09.2016 als Teilanmeldung zu der unter INID-Code 62 erwähnten Anmeldung eingereicht worden.

(54) **DIAGNOSE UND RISIKOSTRATIFIZIERUNG VON HERZINSUFFIZIENZ MITTELS NATRIURETISCHEN PEPTIDEN FÜR NYHA I PATIENTEN**

(57) Die Erfindung betrifft ein Verfahren zur Diagnose und / oder Risikostratifizierung und / oder Outcome-Prognose von Herzinsuffizienz für NYHA I Patienten, wobei eine Bestimmung des Markers proANP, NT-proANP oder jeweils Fragmente oder Teilpeptide davon parallel zu einer Bestimmung von BNP, proBNP und /oder NT-proBNP an zu untersuchenden Patienten durchgeführt wird.

**EP 3 139 173 A1**

**Beschreibung**

Die Erfindung betrifft ein Verfahren zur Diagnose und / oder Risikostratifizierung und / oder Outcome-Prognose von Herzinsuffizienz für NYHA I Patienten, wobei eine Bestimmung des Markers proANP oder Fragmente oder Teilpeptide davon, insbesondere NT-proANP oder Fragmente oder Teilpeptide davon, parallel zu einer Bestimmung von BNP, proBNP und /oder NT-proBNP an zu untersuchenden Patienten durchgeführt wird.

In Europa kommen jährlich etwa eine Million Patienten mit dem Symptom der akuten Atemnot in die Notaufnahmen von Kliniken. Die Atemnot ist ein Leitsymptom vieler Erkrankungen und lässt sich in ca. 35-47% der Fälle auf eine Herzinsuffizienz zurückführen (Januzzi JL Jr, Camargo CA, Anwaruddin S, Baggish AL, Chen AA, Krauser DG, Tung R, Cameron R, Nagurney JT, Chae CU, Lloyd-Jones DM, Brown DF, Foran-Melanson S, Sluss PM, Lee-Lewandrowski E, Lewandrowski KB, The N-terminal Pro-BNP investigation of dyspnea in the emergency department (PRIDE) study, Am J Cardiol. 95(8) (2005), pp. 948-954 und Maisel AS, Krishnaswamy P, Nowak RM, McCord J, Hollander JE, Duc P, Omland T, Storrow AB, Abraham WT, Wu AH, Clopton P, Steg PG, Westheim A, Knudsen CW, Perez A, Kazanegra R, Herrmann HC, McCullough PA; Breathing Not Properly Multinational Study Investigators, Rapid measurement of B-type natriuretic peptide in the emergency diagnosis of heart failure, N Engl J Med. 347(3) (2002), pp. 161-167).

Im Anfangsstadium bemerkt der Patient oft nur wenig von der Herzinsuffizienz. Unbehandelt nimmt die Erkrankung in der Regel an Schwere zu und führt im Spätstadium zu völliger körperlicher Erschöpfung bereits in Ruhe. Die Unterversorgung aller Körperorgane, einschließlich des Herzmuskels selbst, kann in diesem Stadium zum Tode führen. Ist die Erkrankung erst einmal fortgeschritten, ist die Lebenserwartung auch unter optimaler Therapie stark vermindert (ca. 30% Todesfälle pro Jahr). Es ist daher wichtig, eine Herzschwäche möglichst früh zu erkennen und ihre Ursachen konsequent anzugehen.

Um mit einer geeigneten Therapie zu beginnen, bedarf es daher einer frühen Diagnose und Differenzierung der zugrunde liegenden Erkrankung bereits im Frühstadium und in der Not- und Intensivmedizin. Aufgrund unspezifischer Symptome (Luftnot, Husten) ist sowohl die Differenzierung und Abgrenzung der Herzinsuffizienz von anderen Erkrankungen häufig erschwert.

Mittels Bestimmung der Plasmakonzentration des brain natriuretic peptide (BNP bzw. NTproBNP) steht ein Test zur Verfügung, der auch in der Alltagsroutine für die Diagnostik einer Herzinsuffizienz erfolgreich eingesetzt wird (Maisel et al. (supra)). NT-proBNP wird im Stand der Technik zur Verlaufskontrolle von Herzinsuffizienz eingesetzt.

Im Stand der Technik ist ebenfalls bereits proANP als Marker beschrieben. US5498524 beschreibt den Einsatz von proANP zur Diagnose von Herzinsuffizienz bei asymptomatischen Patienten. EP721105B1 beschreibt ein Verfahren zur Bestimmung von proANP mittels geeigneten Antikörpern für Herzerkrankungen.

Es besteht jedoch im Stand der Technik ein hohes Bedürfnis für Patienten mit der Indikation Herzinsuffizienz insbesondere die Diagnose, Risikostratifizierung und outcome-Prognose zu verbessern.

Daher ist es Aufgabe der vorliegenden Erfindung ein Verfahren zur verbesserten Diagnose und / oder Risikostratifizierung und / oder outcome-Prognose von Herzinsuffizienz bereitzustellen.

Nachteilig an bekannten Diagnoseverfahren unter Verwendung der bisher bekannten Markern ist jedoch, dass eine frühzeitige und vollständige Erfassung von Risikopatienten nicht vollständig gelingt und daher eine Risikostratifizierung nur ungenügend erfolgt. Eine der Erfindung weitere zugrunde liegende Aufgabe besteht daher darin, ein Verfahren zur Risikostratifizierung von Herzinsuffizienz zu entwickeln, das eine verbesserte Erfassung von Risikopatienten ermöglicht, dies vor allem für Subgruppen von Patienten.

Überraschender Weise konnte nunmehr gezeigt werden, dass bei der Bestimmung von proANP oder Fragmente oder Teilpeptide davon, insbesondere NT-proANP oder Fragmente oder Teilpeptide davon in paralleler Bestimmung von proBNP, NT-proBNP und / oder BNP eine Verbesserung der Diagnose, Risikostratifizierung und Outcome-Prognose von Herzinsuffizienz und zwar bei einer symptomatischen Herzinsuffizienz ohne Beschwerden (NYHA-Stadium I), insbesondere Linksherzinsuffizienz ohne Beschwerden (NYHA-Stadium I), erreicht werden kann.

Die Aufgabe wird daher durch ein Verfahren nach Anspruch 1 gelöst (nachstehend erfindungsgemäßes Verfahren).

Daher erlaubt das erfindungsgemäße Verfahren besonders vorteilhaft die diagnostische und prognostische Wertigkeit für Patienten der Klasse NYHA I zu verbessern.

In einer besonders bevorzugten Ausführungsform der Erfindung weisen die Patienten zudem einen Body Massindex (BMI) von mindestens 30 kg/m$^2$ auf. Dies ist ein zusätzlicher Parameter der zusätzlich signifikant die diagnostische und prognostische Wertigkeit verbessert.

Im Rahmen dieser Erfindung wird unter "Herzinsuffizienz" ein akutes oder chronisches Unvermögen des Herzens, die Gewebe mit ausreichend Blut und infolge dessen genügend Sauerstoff zu versorgen, um den Gewebestoffwechsel in Ruhe oder unter Belastung sicherzustellen. Klinisch liegt eine Herzinsuffizienz vor, wenn typische Symptome (Dyspnoe, Müdigkeit, Flüssigkeitsretention) bestehen, deren ursächlich eine kardiale Funktionsstörung im Sinne einer systolischen oder diastolischen Funktionsstörung zugrunde liegt. Ebenfalls die chronische Herzinsuffizienz (CHF) ist erfindungsgemäß umfasst(Kardiologie compact, herausgegeben von Christian Mewis, Reimer Riessen und Ioakim Spyridopoulos, 2. unveränderte Auflage, Thieme 2006). Ursachen einer Herzinsuffizienz können sein: Herzklappenfehler (z. B. als Spätfolge des rheumatischen Fiebers), Myokarditis (Herzmuskelentzündung), Herzrhythmusstörungen, Herzinfarkt neben zu hohem Blutdruck (Hypertonie) und/oder Arteriosklerose (Verkalkung) der Herzkranzgefäße (koronare Herzkrankheit). Weiterhin erfindungsgemäß umfasst sind hypertensive Herzkrankheit mit (kongestiver) Herzinsuffizienz, Hypertensive Herz- und Nierenkrankheit mit (kongestiver) Herzinsuffizienz. Die vorliegende Erfindung betrifft solche Patienten die eine Herzinsuffizienz ohne Beschwerden (NYHA-Stadium I) aufweisen. (NYHA = Klassifikation der New York Heart Association (Hoppe UC et al.: Leitlinien zur Therapie der chronischen Herzinsuffizienz. Z Kardiol (2005) 94:488-509)).

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung von Herzinsuffizienz mit dem Ziel eines möglichst günstigen Verlaufs zu ermöglichen. Eine erfindungsgemäße Risikostratifizierung erlaubt in Folge ein effektives Behandlungsverfahren, die betreffend einer Herzinsuffizienz ohne Beschwerden (NYHA-Stadium I) gegeben sind.

Besonders vorteilhaft kann insbesondere in Fällen der Notfall- und /oder Intensivmedizin mittels des erfindungsgemäßen Verfahrens eine sichere Diagnose oder Outcome-Prognose erfolgen. Das erfindungsgemäße Verfahren ermöglicht klinische Entscheidungen, die zu einem schnellen Therapieerfolg führen. Solche klinischen Entscheidungen umfassen ebenfalls weiterführende Behandlungen mittels Arzneimitteln zur Behandlung oder Therapie von Herzinsuffizienz, solche wie ACE-Hemmer, AT1-Antagonisten: Blocker des Angiotensin-II-Rezeptors (Subtyp 1), Betablocker Bisoprolol, Carvedilol, Metoprolol und Nebivolol, Vasopressin-Rezeptor-Antagonisten, Aldosteronantagonisten ab NYHA-Stadium III, Kalzium-Sensitizer (Levosimendan).

In einer weiteren bevorzugten Ausführungsform betrifft das erfindungsgemäße Verfahren daher die Therapiesteuerung einer Herzinsuffizienz ohne Beschwerden (NYHA-Stadium I).

Daher betrifft die Erfindung ebenfalls ein Verfahren zur Risikostratifizierung von Patienten, insbesondere zur Stratifizierung von Patienten für klinische Entscheidungen, vorzugsweise in der zeitlich kritischen Intensivmedizin oder Notfallmedizin und zur Hospitalisierung von Patienten.

In einer weiteren bevorzugten Ausführungsform des erfindungsgemäßen Verfahrens erfolgt die Diagnose zur Prognose, vorzugsweise zur Outcome-Prognose, zur differentialdiagnostischen Früherkennung und Erkennung, zur Beurteilung des Schweregrades und zur therapiebegleitenden Verlaufsbeurteilung für Patienten mit einer Herzinsuffizienz ohne Beschwerden (NYHA-Stadium I).

In einer weiteren Ausführungsform des erfindungsgemäßen Verfahrens wird dem zu untersuchenden Patienten Körperflüssigkeit, insbesondere Blut entnommen, wahlweise Vollblut oder Serum, und die Diagnose erfolgt *in vitro*/*ex vivo,* d.h. außerhalb des menschlichen oder tierischen Körpers. Aufgrund der Bestimmung des Markers proANP oder Fragmente oder Teilpeptide davon, insbesondere NT-proANP oder Fragmente oder Teilpeptide davon und seiner vorhandenen Menge in mindestens einer Patientenprobe kann die Diagnose erfolgen. Parallel wird erfindungsgemäß eine Bestimmung des Markers proBNP, NT-proBNP und / oder BNP in seiner vorhandenen Menge in mindestens einer Patientenprobe vorgenommen.

Im Rahmen dieser Erfindung wird unter "proANP" (auch: NT-proANP) ein freies 98 Aminosäuren enthaltendes Polypeptid/Protein eines atrialen natriuretischen Peptids verstanden oder Fragmente oder Teilpeptide davon. Das N-terminale Fragment proANP (AS 1-98) entsteht aus der Abspaltung des zirkulierenden Hormons alpha-ANP (99-126 mit 28AS) aus dem Prohormon "proANP" (AS 1-126, siehe SEQ ID No. 1), welches aus 126 Aminosäuren besteht und in den Sekretgranula der myoendokrinen Zellen gespeichert wird und erfindungsgemäß umfasst ist. Ferner kann dieses erfindungsgemäße Polypeptid posttranslationale Modifikationen aufweisen, wie Glykolisierung, Lip(o)idisierung oder Derivatisierungen. Insbesondere NT-proANP (AS 1-98) ist überaus stabil im Plasma.

Im Rahmen dieser Erfindung können Fragmente und Teilpeptide insbesondere den midregionalen Bereich der AS 50-90 von NT-proANP (1-98) bzw. proANP (1-126) betreffen und erfindungsgemäß ist insbesondere ein Fragment oder Teilpeptid mit den AS 53-90 des NT-proANP (1-98) bevorzugt (auch: MR-proANP genannt, siehe WO2004046181 und SEQ ID No. 1). Ein geeigneter Assay ist in WO2004046181A1 (BRAHMS AG) offenbart. Weiterhin bevorzugt ist NT-proANP (AS 1-98). Das Fragment (alpha)-ANP (AS 99-126) ist erfindungsgemäß umfasst.

Im Rahmen dieser Erfindung wird unter BNP (AS 77-108) ein B-Typ Natriuretisches Peptid verstanden, welches aus dem Prohormon proBNP (AS 1-108, siehe SEQ ID No. 2) abgespalten wird, wobei zugleich das NT-proBNP (AS 1-76) entsteht. NT-proBNP ist erfindungsgemäß bevorzugt.

"Parallele Bestimmung" bedeutet, dass die Bestimmungen insbesondere simultan oder gleichzeitig erfolgen oder zumindest eine ausreichende Zuordnung oder kombinierte Auswertung erlauben.

In einer weiteren Ausführungsform kann die Bestimmung von proANP, NT-proANP oder jeweils Fragmente oder Teilpeptide davon parallel zu der Bestimmung von BNP, proBNP und / oder NT-proBNP zusätzlich mit weiteren Markern erfolgen und zwar vorzugsweise solche, die bereits auf eine Herzinsuffizienz hinweisen und einen synergetischen Effekt von Markerkombinationen im erfindungsgemäßen Verfahren erlauben.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker, cardiovaskulärer Marker oder ischämischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt sein sowie der cardiovaskuläre Marker aus mindestens einem Marker aus der Gruppe Kreatinkinase, Myeloperoxidase, Copeptin, Myoglobin, kardiale Troponin, CRP ausgewählt sein. Ferner werden hierunter ebenfalls Kreislaufregulierende (Pro)Hormone verstanden, insbesondere wie pro-Gastrin-Releasing Peptid (proGRP), pro-Endothelin (proEnd), pro-Leptin, pro-Neuropeptid-Y, pro-Somatostatin, pro- Neuropeptid-YY, pro-Opiomelanocortin, pro-Adrenomedullin (proADM), Copeptin oder jeweils eine Teilsequenz davon.

Der ischämische Marker kann aus mindestens einem Marker aus der Gruppe Troponin I und T, CK-MB ausgewählt sein.

In einer weiteren Ausführungsform der Erfindung kann das erfindungsgemäße Verfahren mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen an einem Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test), vorzugsweise in Multiparameterbestimmung.

Ein weiterer Erfindungsgegenstand betrifft die Verwendung der Marker proANP oder NT-proANP oder jeweils Fragmente oder Teilpeptide davon parallel zu einer Bestimmung der Marker proBNP, NT-proBNP und / oder BNP an einem zu untersuchenden Patienten zur in-vitro Diagnose und / oder Risikostratifizierung und / oder zur Outcome-Prognose von Herzinsuffizienz ohne Beschwerden (NYHA-Stadium I). MR-proANP ist ein bevorzugtes Fragment oder Teilpeptid. Ebenfalls bezieht sich die erfindungsgemäße Verwendung auf weitere oben genannte Ausführungsformen.

Nachfolgende Beispiele und Figuren dienen zur näheren Erläuterung der Erfindung, jedoch ohne die Erfindung auf diese Beispiele und Figuren zu beschränken.

Beispiele und Figuren:

Beispiel 1: Patienten in NHYA-Klasse 1: Receiver Operator Characteristic (ROC, StatView 5.0 Software für Windows (Abacus, Concepts, Berkley, Canada und MedCalc, Broekstraat, Mariakerke, Belgien)-Analysen für Überleben/Versterben MR-proANP wurde aus dem Plasma der Patienten isoliert und bei -80 Grad Celsius gefroren. Zum Nachweis von MR-proANP wurde ein Sandwich-Assay (LIA) gemäß WO2004046181 der BRAHMS AG verwendet (Morgenthaler et al. Immunoluminometric assay fort he midregion of pro-atrial natriuretic peptide in human plasma, Clin. Chem, 2004; 50: 234-236).

NT-proBNP wurde mit ELICIA (Roche Diagnostics, Penzberg, Deutschland) bestimmt.

Probandenzahl n=66 / 7 Patienten davon sind verstorben.

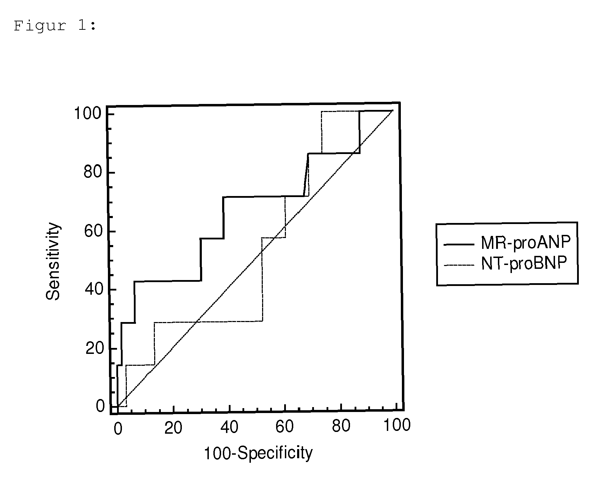

Aus Figur 1 ergibt sich eine Area under the curve (AUC) für MR-proANP = 0,665 und für NT-proBNP = 0,533.

MR-proANP: 0,665 - 0,5 (nicht signifikant): 0,165
NT-proBNP: 0,533 - 0,5 (nicht signifikant): 0,033

$$0,165/0,033 = 5$$

Demnach ergibt sich eine 5-fach gesteigerte diagnostische Wertigkeit (Betrachtung Überleben/Versterben) für MR-proANP im Vergleich zu NT-proBNP in der Subgruppe von Patienten in der NHYA-Klasse 1.

Beispiel 2:

Bestätigung der gesteigerten prognostischen Wertigkeit von MR-proANP im Vergleich zu NT-proBNP mittels Cox-Regression

Univariate Analyse:

Vorgehen: In der Subgruppe der NYHA-1 Patienten wurde für beide Marker (MR-proANP und NT-proBNP) der Median berechnet; für NT-proBNP ergab sich dabei ein 3,7 fach höherer Median als für MR-proANP. Daher wurde in der Cox-Analyse die RiskRatio für NT-proBNP im Vergleich zu MR-proANP für einen 3,7 fach großen Konzentrations-Anstieg berechnet.

Ergebnis der univariaten Cox Proportional Hazard Analyse:

Marker ChiSquare RiskRatio (95% CI) p MR-proANP
(pro 100 pmol/L Anstieg) 6.758 2.517 (1.255-5.047) 0.009 NT-proBNP
(370 pg/mL Anstieg) 0.383 1.107 (0.803-1.525) 0.536

Unabhängig davon, daß sich für NT-proBNP kein signifikantes Ergebnis ergab (p=0,536), verdeutlicht die Risk Ratio von 2.517 für MR-proANP im Vergleich zu NT-proBNP (1.107) eine Verbesserung in der Subgruppe der NYHA-1 Patienten. Eine RiskRatio von 1.000 zeigt an, daß das Risiko zu Versterben pro Konzentrationseinheit nicht ansteigt. Für MR-proANP bedeutet eine RiskRatio von 2.517, daß das Risiko zu Versterben pro Einheit (hier 100 pmoL/L) um 151,7% ansteigt. Im Vergleich zu NT-proBNP ist das ein 14,2 fach höherer Anstieg des Risikos.

Multivariates Modell:

| Marker | ChiSquare | RiskRatio (95% CI) | p |
|---|---|---|---|
| LN MR-proANP | 5,861 | 25,554 (1,854-352,272) | 0,015 |
| LN NT-proBNP | 2,093 | 0,429 (0,136-1,350) | 0,148 |
| Alter | 1,673 | 0,943 (0,863-1,031) | 0,196 |

Das Ergebnis verdeutlicht wieder die Verbesserung im Einsatz von MR-proANP, weil es in einem Modell mit NT-proBNP und Alter als einziger unabhängiger Prädiktor für Versterben in der NYHA-1 Subgruppe bestehen bleibt (p=0,015).

Beispiel 3: Patienten mit einem BMI >=30 kg/m$^2$

Darlegung der Steigerung der prognostischen Wertigkeit von MR-proANP im Vergleich zu NT-proBNP mittels Cox-Regression (Probandenzahl: n=114; 36 Patienten davon sind verstorben)

Univariate Analyse:

Vorgehen: In der Subgruppe der Patienten mit einem BMI >= 30 kg/m$^2$ wurde für beide Marker (MR-proANP und NT-proBNP) der Median berechnet; für NT-proBNP ergab sich dabei ein 5 fach höherer Median als für MR-proANP. Daher wurde in der Cox-Analyse die RiskRatio für NT-proBNP im Vergleich zu MR-proANP für einen 5fach so großen Konzentrations-Anstieg berechnet.

Ergebnis der univariaten Cox Proportional Hazard Analyse:

| Marker MR-proANP | ChiSquare | RiskRatio (95% CI) | p |
|---|---|---|---|
| (100 pmol/L Anstieg) | 14.414 | 1.503 (1.217-1.855) | <0.0001 |
| NT-proBNP (500 pg/mL Anstieg) | 0.662 | 1.024 (0.963-1.094) | 0.416 |

Unabhängig davon, daß sich für NT-proBNP kein signifikantes Ergebnis ergab (p=0,416), verdeutlicht die Risk Ratio von 1.503 für MR-proANP im Vergleich zu NT-proBNP (1.024) Überlegenheit in der Subgruppe der Patienten mit einem BMI >=30. Eine RiskRatio von 1.000 zeigt an, daß das Risiko zu Versterben pro Konzentrationseinheit nicht ansteigt. Für MR-proANP bedeutet eine RiskRatio von 1.503 demnach, daß das Risiko zu Versterben pro Einheit (hier 100 pmoL/L) um 50,3% ansteigt. Im Vergleich zu NT-proBNP ist das ein 21 fach höherer Anstieg des Risikos.

Multivariates Modell:

| Marker | ChiSquare | RiskRatio (95% CI) | p |
|---|---|---|---|
| LN MR-proANP | 7.198 | 2.776 (1.317-5.854) | 0.007 |
| LN NT-proBNP | 0.005 | 0.985 (0.644-1.506) | 0.943 |

Das Ergebnis verdeutlicht wieder die prognostische Steigerung des MR-proANP, weil es in einem Modell mit NT-proBNP als unabhängiger Prädiktor für Versterben in der Subgruppe der Patienten mit einem BMI >=30 bestehen bleibt (p=0,007). NT-proBNP ist dagegen kein unabhängiger Prädiktor.

## Tabelle 1: Übersicht AUC für MR-proANP und NT-proBNP zu verschiedenen Zeitpunkten

| | | 6 months | 12 months | 18 months | 24 months | 36 months | 48 months |
|---|---|---|---|---|---|---|---|
| | Patients at risk | 470 | 406 | 244 | 187 | 144 | 43 |
| | Patients deceased | 55 | 89 | 117 | 129 | 150 | 160 |
| MR-pro ANP | AUC (95% CI) | 0.75 (0.72-0.79) | 0.74 (0.70-0.78) | 0.78 (0.73-0.82) | 0.79 (0.74-0.84) | 0.79 (0.74-0.84) | 0.79 (0.74-0.84) |
| MR-pro ANP | Optimal Cut-Off(pmol/L) | 296 | 296 | 295 | 295 | 267 | 295 |
| MR-pro ANP | Sensitivity (95% CI) | 80.0 (67.0-89.6) | 74.2 (63.6-80.5) | 72.6 (63.6-80.5) | 71.3 (62.7-78.9) | 74.1 (66.0-81.2) | 68.0 (59.9-75.4) |
| MR-pro ANP | Specificity (95% CI) | 63.8 (59.3-68.2) | 66.3 (61.4-70.8) | 71.5 (65.5-77.1) | 74.9 (68.0-80.9) | 71.0 (63.1-78.0) | 77.8 (70.1-84.3) |
| NT-pro BNP | AUC (95% CI) | 0.74 (0.70-0.78) | 0.75 (0.71-0.79) | 0.76 (0.71-0.80) | 0.76 (0.71-0.81) | 0.75 (0.70-0.80) | 0.75 (0.69-0.80) |
| NT-pro BNP | Optimal Cut-Off(pg/mL) | 4083 | 4046 | 4046 | 1770 | 2498 | 2015 |
| NT-pro BNP | Sensitivity (95% CI) | 61.8 (47.7-74.6) | 58.4 (47.5-68.8) | 54.7 (45.2-63.9) | 76.0 (67.7-83.0) | 64.7 (56.2-72.7) | 68.0 (59.9-75.4) |
| NT-pro BNP | Specificity (95% CI) | 80.0 (76.1-83.5) | 82.3 (78.2-85.9) | 85.8 (80.8-89.9) | 64.2 (56.8-71.0) | 74.2 (66.6-80.9) | 69.4 (61.2-76.8) |
| | p-value (between ROCs) | 0.63 | 0.71 | 0.46 | 0.12 | 0.08 | 0.03 |

Patients at risk = Risikopatienten, Patients deceased = Verstorben, Specificity= Spezifität, Sensitivity = Sensitivität, months = Monate

Tabelle 2: Cox Proportional Hazard Analyse für MT-proANP und NT-proBNP zur Vorhersage der Überlebenden in verschiedenen Stadien der Herzinsuffizienz

| Disease Severity | NT-proBNP (per 1000pg/mL increase) (n=774) | | | MR-proANP (per 100pmol/L increase) (n=774) | | | | |
|---|---|---|---|---|---|---|---|---|
| | Chi Square | RR (95%CI) | P | Chi Square | RR (95%CI) | P | Joint Chi Square | Added Prognostic Power * |
| NYHA I (mild) | 0.38 | 1.32 (0.55-3.13) | 0.54 | 6.76 | 2.52 (1.26-5.05) | 0.0093 | 7.16 | 1679% |
| NYHA II&III (moderate) | 70.5 | 1.11 (1.08-1.14) | 0.0001 | 105.0 | 1.19 (1.15-1.23) | 0.0001 | 106.6 | 49% |

(fortgesetzt)

| | NT-proBNP (per 1000pg/mL increase) (n=774) | | | MR-proANP (per 100pmol/L increase) (n=774) | | | | |
|---|---|---|---|---|---|---|---|---|
| NYHA IV (severe) | 5.14 | 1.03 (1.00-1.05) | 0.023 | 6.65 | 1.09 (1.02-1.15) | 0.0099 | 7.77 | 29% |
| *= Zusätzliche prognostische Wertigkeit für MR-proANP Probandenzahl n=774 | | | | | | | | |

Disease Severity = Schweregrad der Erkankung, RR = RiskRatio, increase = Zunahme

Tabelle 3: Klassifikation der New York Heart Association (NYHA)

NYHA I    Keine körperliche Einschränkung. Alltägliche körperliche Belastung verursacht keine inadäquate Erschöpfung, Rhythmusstörungen, Luftnot oder Angina Pectoris.

NYHA II    Leichte Einschränkung der körperlichen Belastbarkeit. Keine Beschwerden in Ruhe. Erschöpfung, Rhythmusstörungen, Luftnot oder Angina pectoris bei alltäglicher körperlicher Belastung.

NYHA III    Höhergradige Einschränkung der körperlichen Leistungsfähigkeit bei gewohnter Tätigkeit. Keine Beschwerden in Ruhe. Erschöpfung, Rhythmusstörungen, Luftnot oder Angina pectoris bei geringer körperlicher Belastung.

NYHA IV    Beschwerden bei allen körperlichen Aktivitäten und in Ruhe. Bettlägerigkeit.

## Patentansprüche

1. Verfahren zur in-vitro Diagnose und / oder Risikostratifizierung und / oder zur Outcome-Prognose von Herzinsuffizienz ohne Beschwerden (NYHA-Stadium I), **dadurch gekennzeichnet,**
dass eine Bestimmung des Markers proANP oder NT-proANP oder jeweils Fragmente oder Teilpeptide davon parallel zu einer Bestimmung des Markers proBNP, NT-proBNP und / oder BNP an einem zu untersuchenden Patienten durchgeführt wird.

2. Verfahren zur in-vitro Diagnose und / oder Risikostratifizierung und / oder zur Outcome-Prognose von Herzinsuffizienz ohne Beschwerden (NYHA-Stadium I) nach Anspruch 1, **dadurch gekennzeichnet, dass** der Marker MR-proANP (AS 53-90 des NT-proANP) ist.

3. Verfahren zur in-vitro Diagnose und / oder Risikostratifizierung und / oder zur Outcome-Prognose von Herzinsuffizienz ohne Beschwerden (NYHA-Stadium I) nach Anspruch 1 oder 2, **dadurch gekennzeichnet, dass** der Patient einen Body Mass-index von mindestens 30 kg/m$^2$ aufweist.

4. Verfahren zur in-vitro Diagnose und / oder Risikostratifizierung und / oder zur Outcome-Prognose von Herzinsuffizienz ohne Beschwerden (NYHA-Stadium I) nach einem der Ansprüche 1 bis 3 zur Durchführung von klinischen Entscheidungen, insbesondere weiterführende Behandlung und Therapie mittels Arzneimitteln, insbesondere in der Intensivmedizin oder Notfallmedizin und zur Hospitalisierung des Patienten.

5. Verfahren zur in-vitro Diagnose und / oder Risikostratifizierung und / oder zur Outcome-Prognose von Herzinsuffizienz ohne Beschwerden (NYHA-Stadium I) nach einem der Ansprüche 1 bis 4, zur differentialdiagnostischen Früherkennung und Erkennung, zur Beurteilung des Schweregrades und zur therapiebegleitenden Verlaufsbeurteilung.

6. Verfahren nach einem der vorherigen Ansprüche,
**dadurch gekennzeichnet,**
dass zusätzlich eine Bestimmung mindestens eines weiteren Marker ausgewählt aus der Gruppe inflammatorischer Marker, cardiovaskulärer Marker oder ischämischer Marker an einem zu untersuchenden Patienten durchgeführt wird.

7. Verfahren nach einem der vorherigen Ansprüche, **dadurch gekennzeichnet, dass** der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt ist.

8. Verfahren nach einem der vorherigen Ansprüche, **dadurch gekennzeichnet, dass** der cardiovaskuläre Marker aus mindestens einem Marker aus der Gruppe Kreatinkinase, Myeloperoxidase, Copeptin, Myoglobin, kardiale Troponin, CRP sowie Kreislaufregulierende (Pro)Hormone, wie pro-Gastrin-Releasing Peptid (proGRP), pro-Endothelin-1, pro-Leptin, pro-Neuropeptid-Y, pro-Somatostatin, pro-Neuropeptid-YY, pro-Opiomelanocortin, pro-Adrenomedullin (proADM), Copeptin oder jeweils eine Teilsequenz davon ausgewählt ist.

9. Verfahren nach einem der vorherigen Ansprüche, **dadurch gekennzeichnet, dass** der ischämischer Marker aus mindestens einem Marker aus der Gruppe Troponin I und T, CK-MB ausgewählt ist.

10. Verfahren nach einem der vorherigen Ansprüche,
**dadurch gekennzeichnet,**
**dass** parallele oder simultane Bestimmungen der Marker durchgeführt werden.

11. Verfahren nach einem der vorherigen Ansprüche,
**dadurch gekennzeichnet,**
**dass** die Bestimmungen an mindestens einer Patientenprobe durchgeführt werden.

12. Verfahren nach einem der vorherigen Ansprüche,
**dadurch gekennzeichnet,**
**dass** die Bestimmungen an einem Analyseautomaten, insbesondere mittels einem Kryptor, durchgeführt werden.

13. Verfahren nach einem der vorherigen Ansprüche,
**dadurch gekennzeichnet, dass** die Bestimmungen mittels einem Schnelltest, insbesondere in Einzel- oder Multiparameterbestimmungen durchgeführt werden.

14. Verwendung der Marker proANP oder NT-proANP oder jeweils Fragmente oder Teilpeptide davon parallel zu einer Bestimmung der Marker proBNP, NT-proBNP und / oder BNP an einem zu untersuchenden Patienten zur in-vitro Diagnose und / oder Risikostratifizierung und / oder zur Outcome-Prognose von Herzinsuffizienz ohne Beschwerden (NYHA-Stadium I).

Figur 1:

**Europäisches Patentamt**
**European Patent Office**
**Office européen des brevets**

# EUROPÄISCHER RECHERCHENBERICHT

Nummer der Anmeldung

EP 16 18 9548

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2006/087373 A (HOFFMANN LA ROCHE [CH]; ROCHE DIAGNOSTICS GMBH [DE]; HESS GEORG [DE];) 24. August 2006 (2006-08-24) * Absatz [0045] - Absatz [0046]; Ansprüche 1-23 * ----- | 1-14 | INV. G01N33/68 G01N33/74 |
| A | WO 2005/124364 A (HOFFMANN LA ROCHE [CH]; ROCHE DIAGNOSTICS GMBH [DE]; HESS GEORG [DE];) 29. Dezember 2005 (2005-12-29) * Seite 35; Anspruch 4; Beispiel 9 * ----- | 1-14 | |
| A,D | WO 2004/046181 A (BRAHMS AG [DE]; BERGMANN ANDREAS [DE]; STRUCK JOACHIM [DE]) 3. Juni 2004 (2004-06-03) * Anspruch 10 * ----- | 1-14 | |
| A | EP 1 577 673 A (HOFFMANN LA ROCHE [CH]; ROCHE DIAGNOSTICS GMBH [DE]) 21. September 2005 (2005-09-21) * Absatz [0036]; Anspruch 3 * ----- | 1-14 | **RECHERCHIERTE SACHGEBIETE (IPC)** |
| A,D | US 5 498 524 A (HALL CHRISTIAN [NO]) 12. März 1996 (1996-03-12) * das ganze Dokument * ----- | 1-14 | G01N |
| A,P | VON HAEHLING ET AL: "Comparison of Midregional Pro-Atrial Natriuretic Peptide With N-Terminal Pro-B-Type Natriuretic Peptide in Predicting Survival in Patients With Chronic Heart Failure", JOURNAL OF THE AMERICAN COLLEGE OF CARDIOLOGY, ELSEVIER, NEW YORK, NY, US, Bd. 50, Nr. 20, 29. Oktober 2007 (2007-10-29), Seiten 1973-1980, XP022332007, ISSN: 0735-1097 * Abbildung 1; Tabelle 5 * ----- | 1-14 | |

Der vorliegende Recherchenbericht wurde für alle Patentansprüche erstellt

| Recherchenort | Abschlußdatum der Recherche | Prüfer |
|---|---|---|
| München | 14. Dezember 2016 | Steinheimer, K |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 16 18 9548

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-12-2016

| Im Recherchenbericht angeführtes Patentdokument | Datum der Veröffentlichung | Mitglied(er) der Patentfamilie | | Datum der Veröffentlichung |
|---|---|---|---|---|
| WO 2006087373 A | 24-08-2006 | CA | 2598582 A1 | 24-08-2006 |
| | | CN | 101120256 A | 06-02-2008 |
| | | EP | 1859283 A1 | 28-11-2007 |
| | | JP | 4828550 B2 | 30-11-2011 |
| | | JP | 2008530571 A | 07-08-2008 |
| | | US | 2008171354 A1 | 17-07-2008 |
| | | WO | 2006087373 A1 | 24-08-2006 |
| WO 2005124364 A | 29-12-2005 | CA | 2567738 A1 | 29-12-2005 |
| | | EP | 1759214 A1 | 07-03-2007 |
| | | ES | 2421556 T3 | 03-09-2013 |
| | | JP | 4838794 B2 | 14-12-2011 |
| | | JP | 2008502888 A | 31-01-2008 |
| | | US | 2007190573 A1 | 16-08-2007 |
| | | WO | 2005124364 A1 | 29-12-2005 |
| WO 2004046181 A | 03-06-2004 | AT | 335763 T | 15-09-2006 |
| | | EP | 1562984 A1 | 17-08-2005 |
| | | ES | 2271674 T3 | 16-04-2007 |
| | | JP | 4656493 B2 | 23-03-2011 |
| | | JP | 2006523298 A | 12-10-2006 |
| | | US | 2006234295 A1 | 19-10-2006 |
| | | WO | 2004046181 A1 | 03-06-2004 |
| EP 1577673 A | 21-09-2005 | AT | 403157 T | 15-08-2008 |
| | | CA | 2500886 A1 | 15-09-2005 |
| | | EP | 1577673 A1 | 21-09-2005 |
| | | ES | 2311895 T3 | 16-02-2009 |
| | | JP | 4343866 B2 | 14-10-2009 |
| | | JP | 4944868 B2 | 06-06-2012 |
| | | JP | 2005274569 A | 06-10-2005 |
| | | JP | 2009080130 A | 16-04-2009 |
| | | US | 2005239138 A1 | 27-10-2005 |
| | | US | 2011229907 A1 | 22-09-2011 |
| US 5498524 A | 12-03-1996 | JP | 3411623 B2 | 03-06-2003 |
| | | JP | H06174718 A | 24-06-1994 |
| | | US | 5498524 A | 12-03-1996 |

EPO FORM P0461

Für nähere Einzelheiten zu diesem Anhang : siehe Amtsblatt des Europäischen Patentamts, Nr.12/82

IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5498524 A
- EP 721105 B1
- WO 2004046181 A
- WO 2004046181 A1

**In der Beschreibung aufgeführte Nicht-Patentliteratur**

- **JANUZZI JL JR ; CAMARGO CA ; ANWARUDDIN S ; BAGGISH AL ; CHEN AA ; KRAUSER DG ; TUNG R ; CAMERON R ; NAGURNEY JT ; CHAE CU.** The N-terminal Pro-BNP investigation of dyspnea in the emergency department (PRIDE) study. *Am J Cardiol.,* 2005, vol. 95 (8), 948-954
- **MAISEL AS ; KRISHNASWAMY P ; NOWAK RM ; MCCORD J ; HOLLANDER JE ; DUC P ; OMLAND T ; STORROW AB ; ABRAHAM WT ; WU AH.** Breathing Not Properly Multinational Study Investigators, Rapid measurement of B-type natriuretic peptide in the emergency diagnosis of heart failure. *N Engl J Med.,* 2002, vol. 347 (3), 161-167

- **CHRISTIAN MEWIS ; REIMER RIESSEN ; IOAKIM SPYRIDOPOULOS.** Kardiologie compact. Thieme, 2006
- **HOPPE UC et al.** *Therapie der chronischen Herzinsuffizienz. Z Kardiol,* 2005, vol. 94, 488-509
- Morgenthaler et al. Immunoluminometric assay fort he midregion of pro-atrial natriuretic peptide in human plasma. *Clin. Chem,* 2004, vol. 50, 234-236

SEQUENCE LISTING

<110>   Brahms GmbH

<120>   Diagnose und Risikostratifizierung von Herzinsuffizienz mittels
        natriuretischen Peptiden für NYHA I Patienten

<130>   BRAHMS 34WOEP-02

<140>   PCT/DE2008/000357
<141>   2008-03-03

<150>   DE10 2007 010 834.8
<151>   2007-03-03

<160>   2

<170>   PatentIn version 3.3

<210>   1
<211>   126
<212>   PRT
<213>   Homo sapiens

<400>   1

Asn Pro Met Tyr Asn Ala Val Ser Asn Ala Asp Leu Met Asp Phe Lys
1               5                   10                  15

Asn Leu Leu Asp His Leu Glu Glu Lys Met Pro Leu Glu Asp Glu Val
            20                  25                  30

Val Pro Pro Gln Val Leu Ser Glu Pro Asn Glu Glu Ala Gly Ala Ala
        35                  40                  45

Leu Ser Pro Leu Pro Glu Val Pro Pro Trp Thr Gly Glu Val Ser Pro
        50                  55                  60

Ala Gln Arg Asp Gly Gly Ala Leu Gly Arg Gly Pro Trp Asp Ser Ser
65                  70                  75                  80

Asp Arg Ser Ala Leu Leu Lys Ser Lys Leu Arg Ala Leu Leu Thr Ala
            85                  90                  95

Pro Arg Ser Leu Arg Arg Ser Ser Cys Phe Gly Gly Arg Met Asp Arg
            100                 105                 110

Ile Gly Ala Gln Ser Gly Leu Gly Cys Asn Ser Phe Arg Tyr
            115                 120                 125

<210>   2
<211>   108
<212>   PRT
<213>   Homo sapiens

<400> 2

His Pro Leu Gly Ser Pro Gly Ser Ala Ser Asp Leu Glu Thr Ser Gly
1                   5                   10                  15

Leu Gln Glu Gln Arg Asn His Leu Gln Gly Lys Leu Ser Glu Leu Gln
            20                  25                  30

Val Glu Gln Thr Ser Leu Glu Pro Leu Gln Glu Ser Pro Arg Pro Thr
            35                  40                  45

Gly Val Trp Lys Ser Arg Glu Val Ala Thr Glu Gly Ile Arg Gly His
        50                  55                  60

Arg Lys Met Val Leu Tyr Thr Leu Arg Ala Pro Arg Ser Pro Lys Met
65                  70                  75                  80

Val Gln Gly Ser Gly Cys Phe Gly Arg Lys Met Asp Arg Ile Ser Ser
                85                  90                  95

Ser Ser Gly Leu Gly Cys Lys Val Leu Arg Arg His
            100                 105